# EUROPEAN PATENT APPLICATION

(11) **EP 4 656 124 A1**
(43) Date of publication of application: **03.12.2025**
(21) Application number: 24315252.7
(22) Date of filing: 29.05.2024
(51) Int. Cl.: A61B 5/00, A61B 5/01, A61B 5/024, A61B 5/0205

(54) **DEVICE INTENDED TO BE WORN BY AN INDIVIDUAL, THE DEVICE BEING CONFIGURED TO DETECT THAT THE INDIVIDUAL IS SUFFERING FROM A HEADACHE**

(71) Applicant: ESSILOR INTERNATIONAL, 94220 Charenton-Le-Pont (FR)
(72) Inventor: Pic, Eléonore, 94300 VINCENNES (FR); Amir, Bruno, 94100 SAINT-MAUR-DES-FOSSES (FR); Sahler, Jean, 92160 ANTONY (FR); Gourraud, Alexandre, 94220 CHARENTON-LE-PONT (FR); Gilbert, Cédric, 92160 ANTONY (FR)
(74) Representative: Korakis-Ménager, Sophie

(57) **Abstract**

A device, head-mountable device, system and computer implemented method, the device being intended to be worn by an individual, the device being configured to detect that the individual is suffering from a headache, the device being further configured to obtain values of a parameter of a vibration of a vessel of the individual, to detect a variation of the values of the parameter of the vibration of the vessel, when the variation is detected, the device is also configured: to obtain a value of a physiological parameter of the individual, to compare the value of the physiological parameter of the individual with a threshold and to detect the headache based on the comparison.

## Description

### Technical field

This disclosure relates to systems and devices intended to be worn by an individual and configured for detecting that the individual is suffering from a headache.

### Background information and prior art

The headache may be migraine headache, tension headache or trigeminal neuralgia.

It could be of interest to detect the beginning of the headache crisis before the pain, possibly during the first minutes/seconds of the headache, for example during the aura crisis of the migraine headache.

This detection allows the notification to the individual of the beginning of the crisis and allows the individual to take actions to minimize the discomfort before the increase of the pain. These actions may be for example applying a filter to avoid photophobia discomfort or taking drug treatment.

There is a need for systems and devices intended to be worn by an individual and configured for detecting that the individual is suffering from a headache.

### Summary

The following presents a simplified summary to provide a basic understanding of various aspects of this disclosure. This summary is not an extensive overview of all contemplated aspects and is intended to neither identify key or critical elements of all aspects nor delineate the scope of any or all aspects. The sole purpose is to present some concepts of one or more aspects in a simplified form as a prelude to the more detailed description that is presented later.

One aspect of this disclosure is a device intended to be worn by an individual, the device being configured to detect that the individual is suffering from a headache, the device being further configured to obtain values of a parameter of a vibration of a vessel of the individual, to detect a variation of the values of the parameter of the vibration of the vessel, when the variation is detected, the device is also configured: to obtain a value of a physiological parameter of the individual, to compare the value of the physiological parameter of the individual with a threshold and to detect the headache based on the comparison.

The device may allow the detection of the beginning of the headache crisis before the pain, possibly during the aura crisis or during the first minutes/seconds of the migraine headache.

This detection allows the notification to the individual of the upcoming crisis. This notification intervenes quickly with an alert and allows the minimization of the discomfort before the pain by proposing one or several of the following solutions:
- To put the right filter on lenses to attenuate symptoms of headache,
- To alert the individual to keep his medication close,
- To alert the individual to avoid driving or dangerous activities and,
- To advise the individual to use light therapy.

Furthermore, this device only needs values of biological parameters of the individual to detect the beginning of the headache crisis. Furthermore, by using sensors in a specific order, the device activates the sensors only when it is necessary and so optimizes its power consumption.

Another aspect of this disclosure is a head-mountable device intended to be worn by an individual, the device being configured to detect that the individual is suffering from a headache, the device being further configured to obtain values of a parameter of a vibration of a vessel of the individual, to detect a variation of the values of the parameter of the vibration of the vessel, when the variation is detected, the device is also configured: to obtain a value of a physiological parameter of the individual, to compare the value of the physiological parameter of the individual with a threshold and to detect the headache based on the comparison.

Another aspect of this disclosure is a system comprising a device, the device being intended to be worn by an individual, the device being configured to detect that the individual is suffering from a headache, the device being further configured to obtain values of a parameter of a vibration of a vessel of the individual, to detect a variation of the values of the parameter of the vibration of the vessel, when the variation is detected, the device is also configured: to obtain a value of a physiological parameter of the individual, to compare the value of the physiological parameter of the individual with a threshold and to detect the headache based on the comparison.

Another aspect of this disclosure is a computer implemented method for detecting that an individual is suffering from a headache. The computer-implemented method comprises obtaining values of a parameter of a vibration of a vessel of the individual detecting a variation of the values of the parameter of the vibration of the vessel. When the variation is detected, the computer-implemented method comprises obtaining a value of a physiological parameter of the individual, comparing the value of the physiological parameter of the individual with a threshold and detecting the headache based on the comparison.

Another aspect of this disclosure is a computer program comprising instructions which, when the computer program is executed by a computer, cause the computer to carry out a method for detecting that an individual is suffering from a headache. The computer-implemented method comprises obtaining values of a parameter of a vibration of a vessel of the individual detecting a variation of the values of the parameter of the vibration of the vessel. When the variation is detected, the computer-implemented method comprises obtaining a value of a physiological parameter of the individual, comparing the value of the physiological parameter of the individual with a threshold and detecting the headache based on the comparison.

A computer may include a memory and a processor. Examples of processors include microprocessors, microcontrollers, graphics processing units (GPUs), central processing units (CPUs), application processors, digital signal processors (DSPs), reduced instruction set computing (RISC) processors, systems on a chip (SoC), field-programmable gate arrays (FPGAs), programmable logic devices (PLDs), state machines, gated logic, discrete hardware circuits, and other suitable hardware configured to perform the various functionality described throughout this disclosure. The memory may be a computer-readable media. By way of example, and not limitation, such computer-readable media may include a random-access memory (RAM), a read-only memory (ROM), an electrically erasable programmable ROM (EEPROM), optical disk storage, magnetic disk storage, other magnetic storage devices, combinations of the aforementioned types of computer-readable media, or any other medium that may be used to store computer executable code in the form of instructions or data structures that may be accessed by the processor of the computer.

Another aspect of this disclosure is a non-transitory program storage device, readable by a computer, tangibly embodying a program of instructions executable by the computer to perform a method for detecting that an individual is suffering from a headache. The computer-implemented method comprises obtaining values of a parameter of a vibration of a vessel of the individual detecting a variation of the values of the parameter of the vibration of the vessel. When the variation is detected, the computer-implemented method comprises obtaining a value of a physiological parameter of the individual, comparing the value of the physiological parameter of the individual with a threshold and detecting the headache based on the comparison.

### Description of the drawings

For a more complete understanding of the description provided herein and the advantages thereof, reference is now made to the brief descriptions below, taken in connection with the accompanying drawings and detailed description, wherein like reference numerals represent like parts.
Figure 1 represents a system with a device intended to be worn by an individual.
Figure 2 represents the different phases of a migraine headache.
Figure 3 represents an eyewear.
Figure 4 represents a removable clip attachable to the eyewear.
Figure 5 represents the method for determining if the individual is suffering from a headache.

### Detailed description of embodiments

The detailed description set forth below in connection with the appended drawings is intended as a description of various possible embodiments and is not intended to represent the only embodiments in which the concepts described herein may be practiced. The detailed description includes specific details for the purpose of providing a thorough understanding of various concepts. However, it will be apparent to those skilled in the art that these concepts may be practiced without these specific details. In some instances, well-known structures and components are shown in block diagram form to avoid obscuring such concepts.

### Description of the system configured for detecting that the individual is suffering from a headache

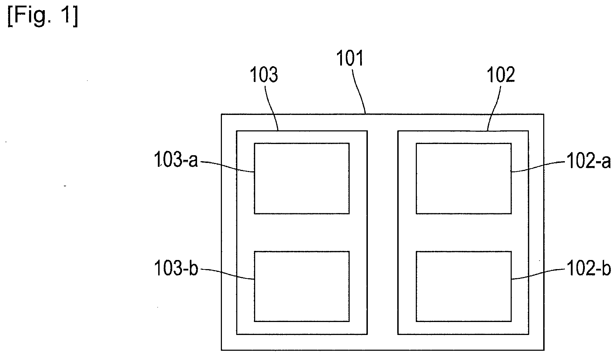

Figure 1 represents a system 101 configured for detecting that the individual is suffering from a headache. The system 101 comprises a calculation module 102 and a device 103 intended to be worn by the individual.

The system 101 may be configured to detect migraine headaches.

The migraine headache is defined as the dilation and inflammation of the brain vessels, especially those of the meninges on the surface of the brain, which can create this typical migraine headache. These dilation and inflammation are caused by the abnormal activation of the trigeminovascular system, which innervates the meningeal vessels and causes nerve stimulation via the release of CGRP, neuropeptides also known as "messengers of pain".

By vessels, one means the components of the circulatory system of the individual that transport blood throughout the body of the individual. The vessels are also known as blood vessels.

Migraine headaches are characterized by repeated seizures mainly manifested by painful headaches. This neurological disease affects 15% of the world's population, mostly women and is very disabling. It is due to abnormal neuronal excitability, linked to complex genetic factors associated with environmental factors. For one in four people suffering frequently from migraine headaches, the severity of the crises has a significant socio-professional impact. Migraine headaches are secondary to the dilation and inflammation of the brain vessels, especially those of the meninges on the surface of the brain and cause pain.

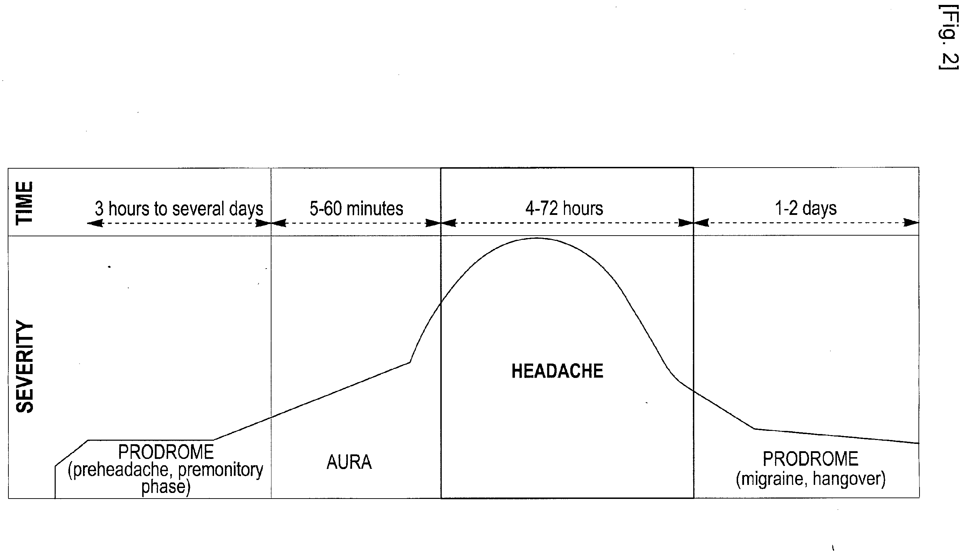

The figure 2 represents the different phase of the migraine headache. The "migraine phase" is composed of the aura and the headache. It is preceded by a phase called prodrome, announcing the arrival of the migraine crisis and is followed by a phase called postdrome.

The device 103 intended to be worn by the individual may be a head-mountable device.

By "head-mountable device", one means a device designed to be mounted on the head of the individual. The head-mountable device may perform several functions, some of which are related to the eyes of the individual.

Examples of head-mountable devices include eyewear, helmets, and clips that can be attached to a cap or an eyewear.

By "eyewear" one means a device designed to be worn in front of one or both eyes of a individual. The eyewear may have a function. The function may be a vision improvement function or a vision correction function. The function may be the protection of the eyes of the individual.

The device 103 may comprise a first module 103-a. The first module 103-a is configured for determining values of a parameter of a vibration of a vessel of the individual.

The parameter of the vibration may be a frequency or an amplitude.

The vessel of the individual may be a temporal vessel of the individual.

The first module 103-a may comprise a first sensor.

The first sensor may be an Inertial Measurement Unit (IMU), a microphone or a piezoelectric sensor.

The device 103 may comprise a second module 103-b. The second module 103-b is configured to determine a value of a physiological parameter of the individual.

The second module 103-b may comprise a second sensor.

The second sensor may be:
- a temperature sensor,
- a set comprising a light emitting device and a photodiode,
- an IMU (identical or different from the one of the first sensor),
- a microphone (identical or different from the one of the first sensor),
- a piezoelectric sensor (identical or different from the one of the first sensor),
- an eye tracker,
- an electric signal variation sensor and/or
- a set of antennas and a dedicated integrated circuit that perform the analog to digital conversion of the signal received by the set of the antennas.

The physiological parameter may be chosen among:
- a heart rate variability of the individual,
- an emission of infra-red by the skin of the individual,
- a change of the spectral properties of the skin, for example the color of the skin in the visible part of the electromagnetic spectrum of the light,
- an evolution of an oxygen saturation (SPO2) of the individual,
- a temperature of the individual and potentially a variation, for example a decrease, of the temperature,
- a dilatation of a pupil of the individual, and
- an intensity of a squint of the individual.

The value of the heart rate variability of the individual, may be determined by the IMU, the microphone, or the piezoelectric sensor.

The value of the infra-red emitted by the skin of the individual may be determined by the set comprising the light emitting device and the photodiode.

The value of the spectral properties of the skin may be determined by the set comprising the light emitting device and the photodiode.

The value of the oxygen saturation (SPO2) of the individual may be determined by the set comprising the light emitting device and the photodiode.

The decrease of the temperature may be caused by vasodilatation, more precisely the vasodilatation of a vessel placed just under a part of a surface of the skin. The value of the temperature may be determined by the temperature sensor. Advantageously the temperature sensor may be placed in the vicinity of the part of the surface of the skin.

The value of the dilatation of the pupil may be determined by the eye tracker.

The value of the intensity of the squint may be determined from a variation in neuromuscular electric signals around the eyes of the individual. This variation may be determined by the set of antennas and the dedicated integrated circuit.

The calculation module 102 comprises a memory 102-a and a processor 102-b.

The calculation module may be a low resource calculation module.

Examples of processors 102-b include microprocessors, microcontrollers, graphics processing units (GPUs), central processing units (CPUs), application processors, digital signal processors (DSPs), reduced instruction set computing (RISC) processors, systems on a chip (SoC), baseband processors, field-programmable gate arrays (FPGAs), programmable logic devices (PLDs), state machines, gated logic, discrete hardware circuits, and other suitable hardware configured to perform the various functionality described throughout this disclosure.

The memory 102-a is computer-readable media. By way of example, and not limitation, such computer-readable media may include a random-access memory (RAM), a read-only memory (ROM), an electrically erasable programmable ROM (EEPROM), optical disk storage, magnetic disk storage, other magnetic storage devices, combinations of the aforementioned types of computer-readable media, or any other medium that may be used to store computer executable code in the form of instructions or data structures that may be accessed by the processor 102-a of the calculation module 102.

The device 103 may comprise the calculation module 102.

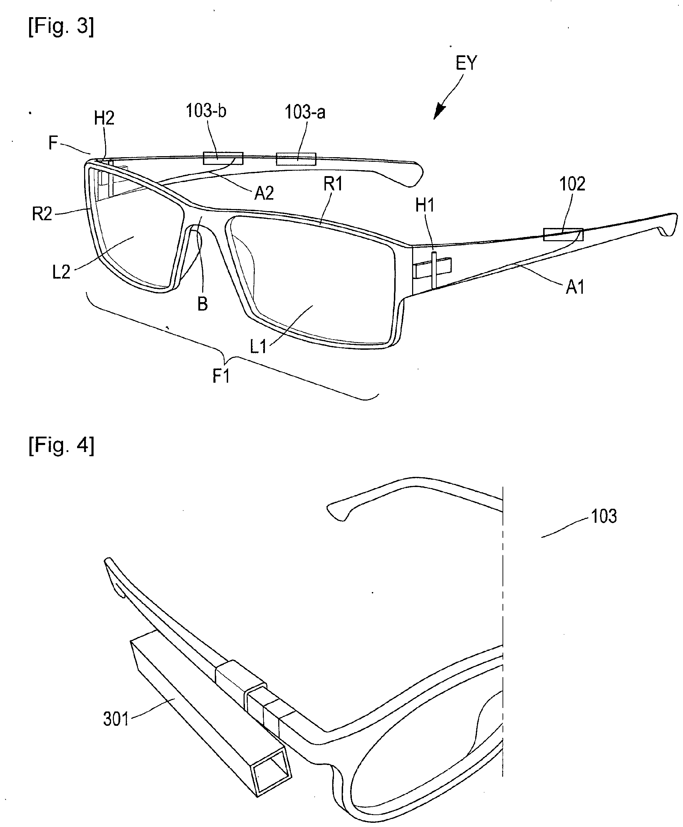

The figure 3 represents the eyewear EY, which is an example of the head-mountable device 103. This eyewear EY comprises two lenses L1 and L2 and a frame F. The frame F comprises two arms or temples A1 and A2 and a front part F1. The front part F1 comprises a right rim R1 and left rim R2 linked together by a bridge B. The front part F1 and the two arms A1 and A2 are linked using two hinges H1 and H2. The hinges H1 and H2 allow the individual to fold the arms A1 and A2 along the front part F1. The rims R1 and R2 of the frame F1 are configured to receive and to maintain the lenses L1 and L2. The eyewear EY can be prescription eyewear for which the correction has been adapted to the individual by an eye care professional and eyewear without correction for example sunglasses.

As represented in the figure 3, the eyewear EY may integrate the calculation module 102, the first module 103-a and the second module 103-b. The calculation module 102, the first module 103-a and the second module 103-b may be located in one or both of the arms A1 and A2 or in the front part F1. When the second sensor is the set of antennas and the dedicated integrated circuit, the antennas may be located in the arm, the hinge or the front part F1 of the eyewear EY.

In other embodiments, represented figure 4, a removable clip 301 may be attached to the eyewear EY. This removable clip 301 may comprise the calculation module 102, the first module 103-a and the second module 103-b.

In other embodiments the calculation module 102 may be integrated in a smartphone, a tracker, or a watch more precisely a wristwatch.

Wristwatches are designed to be worn around the wrist, attached by a watch strap or other type of bracelet, including metal bands, leather straps, or any other kind of bracelet. In this case the watch may include the second module 103-b.

The watch may also be a smartwatch that is a wearable computer in the form of a watch. Modern smartwatches provide generally a local touchscreen interface for daily use, and may comprise sensors used to determine physiological parameters of the individual. The second sensor may be one of these sensors. The calculation module 102 may be included in the smartwatch and may be share with other functions of the smartwatch.

The tracker may be designed to be worn around the wrist, attached by a strap or other type of bracelet, including metal bands, leather straps, or any other kind of bracelet. In this case the watch may include the second module 103-b. The calculation module 102 may also be included in the tracker.

The calculation module 102 may be a computer or may be a virtual machine located on a cloud network or a server not co-located with the subject.

The eyewear EY may also comprise an optical device. This optical device may be an active optical device.

By an active optical device, one means an optical device in which a value of a parameter of a functionality of the optical device can be modified. The value of the parameter may be modified by applying a signal, for example an electrical signal to terminals of the active optical device. The functionality may be one of the following:
- a transmittance level of the active optical device,
- a tint of the active optical device,
- a reflectance level of the active optical device and
- a parameter of a polarization of the active optical device, for example a polarization angle of the active optical device,
- an optical power of the active optical device,
- elements displayed by the active optical device. The elements may be images, symbols, letters, or texts. In embodiments, the elements to be displayed and the position of these elements may be selected.

### Description of the method for detecting that the individual is suffering from a headache 〉

To realize the detection that the individual is suffering from a headache, the memory 102-a may store a computer program comprising instructions which, when the program is executed by the processor 102-b, cause the calculation module 102 to carry out a method, for example a computer implemented method, for detecting that the individual is suffering from a headache.

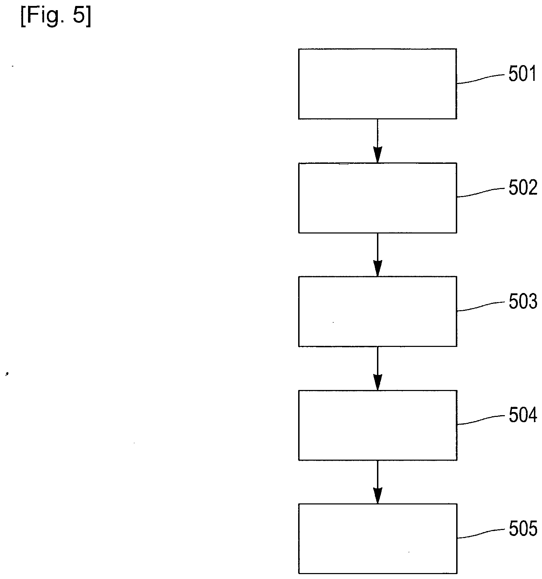

The method, as presented in figure 5, comprises:
- a step 501 of obtaining the values of the parameter of the vibration of the vessel of the individual,
- a step 502 of detecting the variation of the values of the parameter of the vibration of the vessel,
when the variation is detected, the method comprises:
- a step 503 of obtaining the value of the physiological parameter of the individual,
- a step 504 of comparing the value of the physiological parameter of the individual with a threshold and
- a step 505 of detecting the headache based on the comparison.

The values of the parameter of the vibration of the vessel of the individual may be obtained from the first module 103-a.

The value of the physiological parameter of the individual may be obtained from the second module 103-b.

In embodiments the computer, the smartphone, the tracker, the watch more precisely the wristwatch, may be configured to realize at least:
- the step 502 of detecting a variation of the values of the parameter of the vibration of the vessel,
- the step of 504 of comparing the value of the physiological parameter of the individual with a threshold and
- the step 505 of detecting the headache based on the comparison.

Advantageously the physiological parameter may be the heart rate variability of the individual.

To decrease power consumption, one may activate the first module 103-a permanently or with a high frequency and may activate the second module 103-b only once the variation of the values of the parameter of the vibration of the vessel has been detected, or at least with a lower frequency than the frequency of activation of the first module 103-a.

The method may comprise a step of detecting whether the individual is static and when one detects that the individual is static one may realize a step of determining reference values of the parameter of the vibration of the vessel or reference values of the physiological parameters of the individual. In embodiment, the system 101 may be configured to request to the individual to confirm that he does not suffer from headache and to determine the reference values when the individual had confirmed that he does not suffer from headache.

One may determine that the individual is static when he is not dynamic, and one may determine that he is dynamic when he reaches a certain level of activity continuously during a predetermined period. For example, the dynamic threshold value, calculated as a quadratic mean of the acceleration of the three axes of the IMU may be set to 100mg and the predetermined period may be set to 5 min.

For the heart rate variation, the threshold may be the reference value obtained when the individual is static. When the current value of the heart rate variation is lower than the reference of the heart rate variation, one may consider that the individual suffers from headache.

For local temperature, the threshold may be the reference temperature plus 1°C and the threshold for temperature of the body may be 38°C. Therefore, when the local temperature is above the reference temperature plus 1°C or the local temperature of the body at the vessel level is above 38°C one may consider that the individual suffers from headache.

For squint intensity, the threshold may be set to 133% (+33%) of the reference value of the squint intensity. Therefore, when the value of the squint intensity is above 133% of the reference squint intensity, one may consider that the individual suffers from headache.

In embodiments the threshold may be adapted depending on age and genre of the individual.

The threshold may depend on previous values of the physiological parameter.

The threshold may also depend on whether the headache has been previously detected.

The method may also comprise a step of obtaining a value of at least two physiological parameters, a step of comparing the value of the at least two physiological parameters, with at least two thresholds to obtain at least two difference coefficients, a step of determining a global coefficient based on the at least two difference coefficients and at least two ponderation coefficients. The step 505 of detecting the headache may also be based on the global coefficient.

In order to improve the detection of headache one may, after the step 505 of detecting the headache, using a first physiological parameter, confirm the headache using a value of at least one further physiological parameter. The first physiological parameter may be HRV and the further physiological parameter may be the emission of infra-red by the skin, the temperature or the intensity of the squint.

When the first sensor is placed in one of the arms of eyewear EY, it is advantageous to have this arm in contact with the temporal vessel of the individual to detect more accurately the vibration of the temporal vessel.

Moreover, it has been shown in the literature (H.Wang et al., Quantitative Comparison of the Performance of Piezoresistive, Piezoelectric, Acceleration, and Optical Pulse Wave Sensors/ https://doi.org/10.3389%2Ffphys.2019.01563) that the IMU amplitude signal can be correlated with variations of the vibrations of the vessels.

During the first phases of headache there is generally a dilatation of superficial vessel, leading to a modification of the temperature of an area around the vessel and this modification of the temperature may be detected using the temperature sensor (Khan, Yasser & Lochner, Claire & Pierre, Adrien & Arias, Ana. (2015). System Design for Organic Pulse Oximeter. 10.1109/IWASI.2015.7184975.).

The value of the Heart rate variability (HRV) may be determined using the IMU, the microphone or the piezoelectric sensor. Actually, autonomic nervous dysfunction is more prevalent in migraineurs. Heart rate variability (HRV) is a commonly used method to evaluate the cardiac autonomic nervous function modulation, (definition in Malik, M.; Camm, A.J.; Bigger, J.T.; Breithardt, G.; Cerutti, S.; Cohen, R.J.; Coumel, P.; Fallen, E.L.; Kennedy, H.L.; Kleiger, R.E.; et al. Heart rate variability. Standards of measurement, physiological interpretation, and clinical use. Eur. Heart J. 1996, 17, 354-381.). Studies demonstrate that heart rate variation is significantly decreased in episodic migraine (EM) population. (Zhang L, Qiu S, Zhao C, Wang P, Yu S. Heart Rate Variability Analysis in Episodic Migraine: A Cross-Sectional Study. Front Neurol. 2021 Mar 23;12:647092. doi: 10.3389/fneur.2021.647092. PMID: 33833731; PMCID: PMC8021769).

It has been demonstrated in the PCT application WO2022258749 that an IMU located or attached to a frame of the eyewear EY may be used to estimate the heart rate variability.

The eye tracker may detect a slight change in the pupil size. The dilatation of the pupil of the individual is correlated with the level of the subject pain, and therefore to apparition of a migraine. Even in different light conditions, or when the size of the pupil evolves depending on luminance, one may correlated the apparition of a migraine with the dilatation of the pupil of the individual (Hölfe et al, "You can see pain in the eye: Pupillometry as an index of pain intensity under different luminance conditions", International Journal of Psychophysiology, doi : https://doi.org/10.1016/j.ijpsycho.2008.06.008). This detection is particularly accurate when the environment luminance level is at least 100 cd/m.

### Description of actions following the detection of the headache

The system 101 may be configured, when the system 101 detects that the individual is suffering from the headache, to mitigate effects of the headache.

To mitigate the effects of the headache, the system 101 may be configured:
- to adapt a value of a parameter of a functionality of an active optical element of the eyewear EY,
- to alert the individual to keep a medication close,
- to alert the individual to avoid dangerous activities (for example driving) and/or
- to advice the individual to use light therapy.
The adapted functionality of the active element may be the transmittance level of the active optical device or the tint of the active optical device. For example, one may decrease the transmittance level or darken the tint when the headache is detected.
To alert the individual one may use the screen of a smartphone of the individual or one may use the active optical device, configured to display elements, of the eyewear EY. The alert may also be recorded in a daily journal or transmitted to a neurologist.
The light therapy may be realized by the head-mountable device 103 or by an external device.

In other words, the detection, that the individual is suffering from headache, allows the notification of the individual of the upcoming crisis. This notification intervenes quickly with an alert and allows the minimization of the discomfort before the pain by proposing one or several of the following actions:
- To put the right filter on lenses to attenuate symptoms of headache,
- To alert the individual to keep his medication close,
- To alert the individual to avoid driving or dangerous activities and
- To advise the individual to use light therapy.

In embodiments, when the headache is detected by the system 101, the individual may have to confirm, for example using the tracker, the smartphone, the watch more precisely the wristwatch if he feels first signs of the headache.

The data received from the first sensor and the second sensor may be saved to form a database of model signals associated with the indication of the first signs of the headache. This database may comprise data received from different individuals. This database may be filed during tests realized by the individual.

This database may be used to train a machine learning model configured to detect a headache based on signals received from the first sensor and the second sensor.

This database may also be used to determine which sensors can be the most useful for detecting a headache. This can help to optimize battery consumption and increase accuracy of the detection.

The recorded data may also be sent to a neurologist for medical examination, more precisely the sent date may be:
- The frequency, duration of the crisis,
- The pattern can be associated with particular behavior of the individual,
- The sensor signature can indicate a type of headache (migraine headache due to hypertension, hormones, etc.).

## Claims

1. A device (103) intended to be worn by an individual, the device (103) being configured to detect that the individual is suffering from a headache,
the device (103) being further configured:
• to obtain values of a parameter of a vibration of a vessel of the individual,
• to detect a variation of the values of the parameter of the vibration of the vessel,
when the variation is detected, the device (103) is also configured:
• to obtain a value of a physiological parameter of the individual,
• to compare the value of the physiological parameter of the individual with a threshold and
• to detect the headache based on the comparison.

2. The device (103) according to the claim 1,
the vessel of the individual being a temporal vessel of the individual.

3. The device (103) according to the claim 1 or 2,
the device (103) also comprising an IMU, a microphone or a piezoelectric sensor, for obtaining the values of the parameter of the vibration of the vessel.

4. The device (103) according to any one of the claims 1 to 3,
the device (103) being configured to detect whether the individual is static and when the device (103) detects that the individual is static the device (103) being configured to detect the variation of the average of the vibration of the vessel.

5. The device (103) according to any one of the claims 1 to 4,
the physiological parameter being chosen among:
• a heart rate variability of the individual,
• a temperature of the individual and potentially a variation of the temperature and
• an intensity of a squint of the individual.

6. The device (103) according to any one of the claims 1 to 5,
the device (103) also comprising, for obtaining the value of the physiological parameter, a temperature sensor and/or a set comprising a light emitting device and a photodiode.

7. The device (103) according to any one of the claims 1 to 6,
the threshold depending on previous values of the physiological parameter.

8. The device (103) according to any one of the claims 1 to 7,
the threshold depending also on whether the headache has been previously detected.

9. The device (103) according to any one of the claims 1 to 8,
the device (103) being configured to obtain a value of at least two physiological parameters, the device (103) being configured to compare the value of the at least two physiological parameters, with at least two thresholds to obtain at least two difference coefficients,
the device (103) being also configured to determine a global coefficient based on the at least two difference coefficients and at least two ponderation coefficients,
the device (103) being configured to detect the headache also based on the global coefficient.

10. Head mountable device (103) intended to be worn by an individual, the head mountable device (103) being configured to detect that the individual is suffering from a headache,
the head mountable device (103) being further configured:
• to obtain values of a parameter of a vibration of a vessel of the individual,
• to detect a variation of the values of the parameter of the vibration of the vessel,
when the variation is detected, the head mountable device (103) is also configured:
• to obtain a value of a physiological parameter of the individual,
• to compare the value of the physiological parameter of the individual with a threshold and
• to detect the headache based on the comparison.

11. The head mountable device (103) according to the claim 10,
the head mountable device (103) also comprising an IMU, a microphone or a piezoelectric sensor, for obtaining the values of the parameter of the vibration of the vessel.

12. System (101) comprising a head mountable device (103), the head mountable device (103) being intended to be worn by an individual, the system (101) being configured to detect that the individual is suffering from a headache,
the system (101) being further configured:
• to obtain values of a parameter of a vibration of a vessel of the individual,
• to detect a variation of the values of the parameter of the vibration of the vessel,
when the variation is detected, the system (101) is also configured:
• to obtain a value of a physiological parameter of the individual,
• to compare the value of the physiological parameter of the individual with a threshold and
• to detect the headache based on the comparison.

13. The system (101) according to the claim 12,
the system (101) being configured, when the system (101) detects that the individual is suffering from the headache, to mitigate effects of the headache.

14. The system (101) according to the claim 13,
to mitigate the effects of the headache, the system (101) being configured:
- to adapt a value of a parameter of a functionality of an active optical element of the head mountable device (103),
- to alert the individual to keep a medication close,
- to alert the individual to avoid dangerous activities and/or
- to advice the individual to use light therapy.

15. Computer-implemented method for detecting that an individual is suffering from a headache,
the computer-implemented method comprising:
• obtaining (501) values of a parameter of a vibration of a vessel of the individual,
• detecting (502) a variation of the values of the parameter of the vibration of the vessel,
when the variation is detected, the computer-implemented method comprising:
• obtaining (503) a value of a physiological parameter of the individual,
• comparing (504) the value of the physiological parameter of the individual with a threshold and
• detecting (505) the headache based on the comparison.
